(19) Europäisches Patentamt
European Patent Office
Office européen des brevets

(11) EP 1 264 176 B1

(12) **EUROPEAN PATENT SPECIFICATION**

(45) Date of publication and mention of the grant of the patent:
**19.01.2005 Bulletin 2005/03**

(21) Application number: **01917974.6**

(22) Date of filing: **16.03.2001**

(51) Int Cl.$^7$: **G01N 33/497**, A01C 1/02, C12M 1/34

(86) International application number:
**PCT/NL2001/000217**

(87) International publication number:
**WO 2001/069243 (20.09.2001 Gazette 2001/38)**

# (54) MEASURING METABOLIC RATE CHANGES

MESSUNG VON STOFFWECHSELÄNDERUNGEN

MESURE DE CHANGEMENTS D'ACTIVITE METABOLIQUE

(84) Designated Contracting States:
**AT BE CH CY DE DK ES FI FR GB GR IE IT LI LU MC NL PT SE TR**

(30) Priority: **17.03.2000 EP 00200990**

(43) Date of publication of application:
**11.12.2002 Bulletin 2002/50**

(73) Proprietor: **Nederlandse Organisatie voor Toegepast-Natuurwetenschappelijk Onderzoek TNO**
**2628 VK Delft (NL)**

(72) Inventors:
- **VAN DUIJN, Albert**
  **NL-2341 BV Oegstgeest (NL)**
- **KÖNIG, Johan, Willem**
  **NL-2201 VE Noordwijk (NL)**

(74) Representative:
**Prins, Adrianus Willem, Mr. Ir. et al**
**Vereenigde,**
**Nieuwe Parklaan 97**
**2587 BN Den Haag (NL)**

(56) References cited:
**EP-A- 0 448 923**
**WO-A-96/36875**
**WO-A-98/12348**
**WO-A-98/54354**
**US-A- 5 863 460**

- **AN L ET AL: "A NEW BIOSENSOR FOR RAPID OXYGEN DEMAND MEASUREMENT" WATER ENVIRONMENT RESEARCH,US,WATER ENVIRONMENT FEDERATION, ALEXANDRIA, vol. 70, no. 5, 1 July 1998 (1998-07-01), pages 1070-1074, XP000793304 ISSN: 1061-4303**
- **COX M E ET AL: "DETECTION OF OXYGEN BY FLUORESCENCE QUENCHING" APPLIED OPTICS,US,OPTICAL SOCIETY OF AMERICA,WASHINGTON, vol. 24, no. 14, 1985, pages 2114-2120, XP000885259 ISSN: 0003-6935 cited in the application**
- **GIDROL X ET AL: "ACCUMULATION OF REACTIVE OXYGEN SPECIES AND OXIDATION OF CYTOKININ IN GERMINATING SOYBEAN SEEDS" EUROPEAN JOURNAL OF BIOCHEMISTRY,DE,BERLIN, vol. 224, no. 1, 15 August 1994 (1994-08-15), pages 21-28, XP000610708 ISSN: 0014-2956**
- **DATABASE WPI Week 199213 Derwent Publications Ltd., London, GB; AN 1992-103639 XP002147978 & SU 1 645 893 A (REVUT V I), 30 April 1991 (1991-04-30)**
- **YEOUNG Y.R.: 'Effects of Water Potential and Oxygen Concentration on Respiration during Muskmelon Seed Priming' J. KOR. SOC. HORT. SCI. vol. 36, no. 6, 1995, KOREA, pages 774 - 779**
- **BUJALSKI W. ET AL: 'Scale-up Studies for Osmotic Priming and Drying of Carrot Seeds' AGRIC. ENGNG. RES. vol. 48, no. 4, 1991, UK, pages 287 - 302**
- **BAKER A.M.; HATTON W.: 'Calcium peroxide as a seed coating material for padi rice: I. Requirement for and provision of oxygen' PLANT AND SOIL vol. 99, no. 2-3, 1987, NL, pages 357 - 363**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- CHUGH L.K.; SAWHNEY S.K.: 'Effect on Cadmium on Germination, Amylases and Rate of Respiration of Germinating Pea Seeds' ENVIRONMENTAL POLLUTION vol. 92, no. 1, 1996, pages 1 - 5
- BARNIKOL W.K.R. ET AL: 'An Innovative Procedure for the Detection of Oxygen Based on Luminescence Quenching, for Use in Medicine, Biology, Environmental Research and Biotechnology' BIOMEDIZINISCHE TECHNIK vol. 41, no. 6, 1996, pages 170 - 177

## Description

The invention relates to methods for measuring metabolic states, metabolic rates and changes therein of seeds.

Biological activities of organisms are manifold and are often studied by the chemical, physical, physiological or morphological ways they manifest themselves. Culturing organisms requires managing these biological activities, and managing these activities often requires measuring the underlying metabolic activities. Here, germination of seeds is discussed.

A new plant formed by sexual reproduction starts as an embryo within the developing seed, which arises from the ovule. When mature, the seed is the means by which the new individual is dispersed, though frequently the ovary wall or even extrafloral organs remain in close association to form a more complex dispersal unit as in grasses and cereals. The seed, therefore, occupies a critical position in the life history of the higher plant. The success with which the new individual is established -the time, the place, and the vigour of the young seedling- is largely determined by the physiological and biochemical features of the seed. Of key importance to this success are the seed's responses to the environment and the food reserves it contains, which are available to sustain the young plant in the early stages of growth before it becomes an independent, autotrophic organism, able to use light energy. People also depend on these activities for almost all of their utilisation of plants.

Cultivation of most crop species depends on seed germination, though, of course, there are exceptions when propagation is carried out vegetatively. Moreover, seed such as those of cereals and legumes are themselves major food sources whose importance lies in the storage reserves of protein, starch, and oil laid down during development and maturation.

In the scientific literature the term germination is often used loosely and sometimes incorrectly and so it is important to clarify its meaning. Germination begins with water uptake by the seed (imbibition) and ends with the start of elongation by the embryonic axis, usually the radicle. It includes numerous events, e.g., protein hydration, subcellular structural changes, respiration, macromolecular syntheses, and cell elongation, none of which is itself unique to germination. But their combined effect is to transform an organism having a dehydrated, resting metabolism into an organism having an active metabolism, culminating in growth.

Germination sensu stricto therefore does not include seedling growth, which commences when germination finishes. Hence, it is incorrect, for example, to equate germination with seedling emergence from soil since germination will have ended sometime before the seedling is visible. Seed testers often refer to germination in this sense because their interests lie in monitoring the establishment of a vigorous plant of agronomic value. However, physiologists do not encourage such a definition of the term germination but in general acknowledge its widespread use by seed technologists. It would however be preferable to find a more defined definition. Processes occurring in the nascent seedling, such as mobilisation of the major storage reserves, are also not part of germination: they are postgermination events.

A seed in which none of the germination processes is taking place is said to be quiescent. Quiescent seeds are resting organs, generally having a low moisture content (5-15%) with metabolic activity almost at a standstill. A remarkable property of seeds is that they are able to survive in this state, often for many years, and subsequently resume a normal, high level of metabolism. For germination to occur quiescent seeds generally need only to be hydrated under conditions that encourage metabolism, e.g., a suitable temperature and presence of oxygen.

Components of the germination process, however, may occur in a seed that does not achieve radicle emergence. Even when conditions are apparently favourable for germination so that imbibition, respiration, synthesis of nucleic acids and proteins, and a host of other metabolic events all proceed, culmination in cell elongation does not occur, for reasons that are still poorly understood; such a seed expresses dormancy. Seeds that are dispersed from the parent plant already containing a block to the completion of germination show primary dormancy. Sometimes, a block(s) to germination develops in hydrated, mature seeds when they experience certain environmental conditions, and such seeds show induced or secondary dormancy. Dormant seeds are converted into germinable seeds (i.e., dormancy is broken) by certain "priming" treatments such as a light stimulus or a period at low or alternating temperature which nullify the block to germination but which themselves are not needed for the duration of germination process.

The extent to which germination has progressed can be determined roughly, say by measuring water uptake or respiration, but these measurements give us only a very broad indication of what stage of the germination process has been reached. No universally useful biochemical marker of the progress of germination has been found. The only stage of germination that we can time fairly precisely is its termination. Emergence of the axis (usually the radicle) from the seed normally enables us to recognise when germination has gone to completion, though in those cases where the axis may grow before it penetrates through the surrounding tissues, the completion of germination can be determined as the time when a sustained rise in fresh weight begins.

We are generally interested in following the germination behaviour of large numbers of seeds, e.g., all the seeds produced by one plant or inflorescence, or all those collected in a soil sample, or all those subjected to certain experimental treatment. The degree to which germination has been completed in a population is usually expressed as a percentage, normally determined at time intervals over the course of the germination period which can be expressed in so-called germination curves, about which some general points should be made. Germination curves are usually sigmoidal, a minority of the seeds in the population germinates early, then the germination percentage increases more or less rapidly, and finally few late germinatores emerge. The curves are often positively skewed because a greater percentage germinates in the first half of the germination period than in the second. But although the curves have the same general shape, important differences in behaviour between populations are evident. For example, curves often flatten off when only a low percentage of the seeds has germinated, showing that this population has low germination capacity, i.e., the proportion of seeds capable of completing germination is low. Assuming that these seeds are viable, the behaviour of the population could be related to dormancy or to environmental conditions, such as temperature or light, which do not favour germination of most of the seeds.

The shape of the curves also depends on the uniformity of the population, i.e., the degree of simultaneity or synchrony of germination. When a limited percentage of seeds succeeds in germinating fairly early, but the remainder begin to do so only after a delay the population seems to consists of two discrete groups: the quick and the slow germinators. This example also illustrates the point that populations with the same germination capacity can differ in other respects.

Three respiratory pathways are assumed to be active in the imbibed seed: glycolysis, the pentose phosphate pathway, and the citric acid (Krebs or tricarboyxlic acid) cycle. Glycolysis, catalysed by cytoplasmic enzymes, operates under aerobic and anaerobic condition to produce pyruvate, but in the absence of $O_2$ this is reduced further to ethanol, plus $CO_2$, or to lactic acid if no decarboxylation occurs. Anaerobic respiration, also called fermentation, produces only two ATP molecules per molecule of glucose respired, in contrast to six ATPs produced during pyruvate formation under aerobic conditions. In the presence of $O_2$, further utilisation of pyruvate occurs within the mitochondrion: oxidative decarboxylation of pyruvate produces acetyl-CoA, which is completely oxidised to $CO_2$ and water via the citric acid cycle to yield up to a further 30 ATP molecules per glucose molecule respired. The generation of ATP molecules occurs during oxidative phosphorylation when electrons are transferred to molecular $O_2$ along an electron transport (redox) chain via a series of electron carriers (cytochromes) located on the inner membrane of the mitochondrion. An alternative pathway for electron transport, which does not involve cytochromes, may also operate in mitochondria.

The pentose phosphate pathway is an important source of NADPH, which serves as a hydrogen and electron donor in reductive biosynthesis, especially of fatty acid. Intermediates in this pathway are starting compounds for various biosynthetic processes, e.g., synthesis of various aromatics and perhaps nucleotides and nucleic acid. Moreover, complete oxidation of hexose via the pentose phosphate pathway and the citric acid cycle can yield up to 29 ATPs.

Respiration by mature "dry" seeds (usual moisture content: 10-15%) of course is extremely low when compared with developing or germinating seeds, and often measurements are confounded by the presence of a contaminating microflora. When dry seeds are introduced to water ,there is an immediate release of gas. This so-called "wetting burst" which may last for several minutes, is not related to respiration, but is the gas that is released from colloidal adsorption as water is imbibed. This gas is released also when dead seeds or their contents, e.g., starch, are imbibed.

Keto acids (e.g.; α-ketoglutarate, pyruvate), which are important intermediates in respiratory pathways, are chemically unstable and may be absent from the dry seed. A very early metabolic event during imbibition, occurring within the first few minutes after water enters the cells, is their reformation from amino acids by deamination and transamination reactions (e.g., of glutamic acid and alanine).

The consumption of $O_2$ by many seeds follows a basic pattern although the pattern of consumption by the embryo differs ultimately from that by storage tissues. Respiration is considered to involve three or four phases:

Phase 1. Initially there is a sharp increase in $O_2$ consumption, which can be attributed in part to the activation and hydration of mitochondrial enzymes involved in the citric acid cycle and electron transport chain. Respiration during this phase increases linearly with the extent of hydration of the tissue.

Phase 2. This is characterised by a lag in respiration as $O_2$ uptake is stabilised or increases only slowly. Hydration of the seed parts is now completed and all pre-existing enzymes are activated. Presumably there is little further increase in respiratory enzymes or in the number of mitochondria during this phase. The lag phase in some seeds may occur in part because the coats or other surrounding structures limit $O_2$ uptake to the imbibed embryo or storage tissues, leading temporarily to partially anaerobic conditions. Removal of the testa from imbibed pea seeds, for example, diminishes the lag phase appreciably. Another possible reason for this lag is that the activation of the glycolytic pathway during germination is more rapid than the development of mitochondria. This could lead to an accumulation of pyruvate because of deficiencies in the citric acid cycle or oxidative phosphorylation (electron transport chain); hence, some pyruvate would be diverted temporarily to the fermentation pathway, which is not $O_2$ requiring.

Between phase 2 and 3 in the embryo the radicle penetrates the surrounding structures: germination is completed.

Phase 3. There is now a second respiratory burst. In the embryo, this can be attributed to an increase in activity of newly synthesised mitochondria and respiratory enzymes in the proliferating cells of the growing axis. The number of mitochondria in storage tissues also increases, often in association with the mobilisation of reserves. Another contributory factor of the rise in respiration in both seeds parts could be an increased $O_2$ supply through the now punctured testa (or other surrounding structures).

Phase 4. This occurs only in storage tissues and coincides with their senescence following depletion of the stored reserves.

The lengths of phases 1-4 vary from species to species owing to such factors as differences in rates of imbibition, seed-coat permeability to oxygen, and metabolic rates. Moreover, the lengths of the phases will vary considerably with the ambient conditions, especially the temperature. In a few seeds, e.g., Avena fatua, there is no obvious lag phase in oxygen uptake. The reasons for its absence are not known, but it could be because efficient respiratory systems become established early following imbibition, including the development of newly active mitochondria, thus ensuring a continued increase in $O_2$ utilisation. Also, coat impermeability might not restrict $O_2$ uptake prior to the completion of germination.

During germination a readily available supply of substrate for respiration must be present. This may be provided to a limited extent by hydrolysis of the major reserves, e.g., triacylglycerols, which are present in almost all parts of the embryo, including the radicle and hypocotyl, although their greatest concentration is in storage tissues. It is important to note, however, that extensive mobilisation of reserves is a postgerminative event. Most dry seeds contain sucrose, and many contain one or more of the raffinose-series oligosaccharides: raffinose (galactosyl sucrose), stachyose (digalactosyl sucrose), and verbascose (trigalactosyl sucrose), although the latter is usually present only as a minor component. The distribution and amounts of these sugars within seeds are very variable, even between different varieties of the same species.

During germination, sucrose and the raffinose-series oligosaccharides are hydrolysed, and in several species the activity of α-galactosidase, which cleaves the galactose units from the sucrose, increases as raffinose and stachyose decline. Although there is little direct evidence that the released monosaccharides are utilised as respiratory substrates, there is strong circumstantial evidence. Free fructose and glucose may accumulate in seeds during the hydrolysis of sucrose and the oliosaccharides, but there is no buildup of galactose (e.g., in mustard, Sinapis alba). Hence, it is probably rapidly utilised, perhaps through incorporation into cell walls or into galactolipids of the newly forming membranes in the cells of developing seedling.

Virtually all metabolic pathways in living organisms, and not only those related to germination, relate to the uptake or release of metabolic gasses, of which the two most important are oxygen and carbon dioxide; examples of others are carbon mono-oxide, nitric oxide, nitric dioxide, dinitric oxide, ethylene and ethanol. Classical is the way it could be demonstrated that oxygen is central to life. A mouse, placed under a glass bulb together with a burning candle, died when the flame dwindled and died, showing that also the mouse could not do without the oxygen. Undoubtedly, the level of carbon dioxide in the glass bulb was, as a consequence, high.

The above example illustrates an archaic way of measuring the underlying metabolic activity of an organism. More modern methods have been developed which comprise measuring oxygen or other metabolic gasses in gas or liquid media. Oxygen, or other gasses, in gas are often measured by analysis with gas-chromatography. In liquid gas contents are often measured by flushing some liquid through an electro-chemical measurement device.

For both types of measurements the sample is in general consumed and cannot be reused for other measurements. This has a number of further disadvantages: A container with the organism under study has to be opened for a gas determination, which may disrupt the activities to be measured or otherwise hinder accurate determination. Also, for each point in a time series different samples are necessary for which the container has to be opened again. Normally this makes the number of samples very large and does not allow for using small containers to begin with. Furthermore, sample to sample variability makes it very difficult to get reliable figures and the costs for handling and measuring a sample are in general very high.

Other known methods for monitoring metabolic gases use Gilson respirometers or polarographic electrodes (7, 8).

A method according to the invention is performed in a confined container which may have different sizes and/or shapes.

A confined container (also called confined space; the terms may be used interchangeably herein) is herein defined as a container that is properly shut to (essentially) avoid gas exchange between the confined container and the surrounding and furthermore a confined container is defined as a container that is essentially not opened during measurements but to which additional substances (oxygen, nutrients, growth hormones, etc.) can be added with for example a valve or injection system. Because the container is essentially not opened all changes in a metabolic gas concentration are attributed to the metabolic state or rate or a change therein of the seed which is located in the container. changes in gas concentration must be attributed to the production and/or consumption of a metabolic gas, thus of life, or at least in a state of life-like activity.

The seed is brought in a small confined container, along with some water to induce the germination process.

Seeds can of course be totally immersed in water, which typically allows for measurements to be made in the liquid but usually measurement of the air or gas above the seed will be sufficient. Due to the germination at a certain point in time the seed will start to consume oxygen and produce carbon dioxide. The oxygen concentration will drop from the moment the germination starts and the carbon dioxide concentration will rise. The gas concentration is measured optically. This can for example be achieved by a measuring device which is at least partly set up within the confined container, but measurements can also be made through a clear portion of the wall of the confined container, which for example could be made of glass.

The optical method is based on fluorescence quenching of fluorescent compounds by oxygen (1-4, 9), to determine the oxygen levels inside a container, without opening it. A sample can be measured over and over again in the time, and is not destroyed. Moreover, because the sample is not destroyed the number of samples necessary to do a time study is considerably lower compared to conventional methods. The fluorescent dye is a suitable organo-metal, present in said confined container. An oxygen sensitive dye such as a ruthenium bipyridyl complex, or Tris-$Ru^{2+}$ 4,7 biphenyl 1,10 phenantrolin; or another Ru(ruthenium)-complex, or another organo-metal complex, such as an Os-complex or a Pt-complex, is suitable.

The optical oxygen sensing measurement technique used herein is based on the fluorescence quenching of a metal organic fluorescent dye. The dye which is very sensitive to oxygen, is for example excited by a short laser light-pulse of for example 1 microsecond. After the excitation has stopped the oxygen sensitive dye emits fluorescent light with a decay curve which depends on the oxygen concentration. The process behind this phenomenon is called dynamic quenching.

Preferably said dye is present in a gas permeable compound such as silica or a hydrophobic polymer such as a (optionally fluoridated) silicone polymer, in PDMS (polydimethylsiloxane), in PTMSP (polytrimethylsilylpropyl), or in a mixture thereof but of course it can be contained in other suitable compounds as well. In a preferred embodiment the dye is present in at least a part of an inner coating of said confined container, for example situated on the inside of an optically transparent part of the confined container when measurement is from the outside.

Measuring can be achieved by measuring the fluorescence lifetime. The excited molecules are deactivated by oxygen in a collision process. The quenching process does not consume the oxygen so liquid medium does not necessarily have to be stirred to obtain the measurements. The fluorescence lifetime gets shorter because the probability of the molecules to be deactivated gets higher for molecules which stay longer in the excited state. The effect is proportional with the quencher concentration. The relation between fluorescence lifetime and oxygen concentration is given by the Stern Volmer equation (1)

$$\frac{\tau_0}{\tau} = 1 + C_{SV}{*}[o_2]$$

where $\tau_0$ is the fluorescence lifetime at quencher ($O_2$) concentration zero, $\tau$ is the fluorescence lifetime at a specific quencher ($O_2$) concentration. Csv is the Stern-Volmer constant and $[O_2]$ is the gas concentration.

Measuring can also be achieved by measuring the fluorescence intensity. The fluorescent compound is excited by a continuously radiating light source such as a LED and the fluorescence intensity is measured. More oxygen caused less fluorescence. The relation between the oxygen concentration and the intensity is given by the Stern Volmer equation (2)

$$\frac{I_0}{I} = 1 + C_{SV}{*}[o_2]$$

where $I_0$ is the fluorescence intensity at quencher ($O_2$) concentration zero, I is the fluorescence intensity at a specific quencher ($O_2$) concentration. Csv is the Stern-Volmer constant and $[O_2]$ is the gas concentration.

Using the fluorescence lifetime method has the advantage that the measurement is independent of the source intensity, detector efficiency, fluorescent probe concentration etc. A method based on this principle is robust and less prone to drift. Moreover, because the quenching process does not consume oxygen, the method as provided by the invention is very useful to measure metabolic rate changes of seeds by measuring an increase or decrease in oxygen consumption.

A method as provided by the invention is based on a time gated measurement (Fig.1). In this measurement method the fluorescence is determined in two time windows (A and B) after a light pulse. Fluorescence lifetime is a function of the ratio between A and B and is proportional to the oxygen concentration. The person skilled in the art is aware of the huge array of possible experimental set-ups. Figure 2 shows an example of a simplified experimental set-up. In this simplified set-up the confined container (having possibly different sizes and/or shapes) contains an oxygen sensitive coating situated on the inside of an optically transparent part of the confined container. Another possibility is to provide the oxygen sensitive substance to the material from which the confined contain is made. Yet another possibility is to place the oxygen sensitive substance via a holder at any desired position within the confined container. The oxygen sensitive substance can be placed at every desired position as long as it is possible to reach the position with for example a laser to provoke excitation and to determine the fluorescence signal with a detector. Detection of the fluorescence is made visible by for example a measuring device or with help of a computer and suitable computer programs. In the above described simplified set-up the confined container is not physically part of the measuring device, but is clear that it possible to set-up a measuring device which is partly set-up in a confined container. A confined container can have different shapes and/or sizes and can be made of different materials as long as it is possible to perform measurements through a clear portion of the wall of the confined container, which for example could be made of glass. As described it is also possible that part of measuring device is part of the confined container in which case it is not necessary for the wall of the container to be clear. Figure 3 shows a more detailed instrumental set up. A light source (e.g. LED or laser) is pulsed, the light pulses are filtered and excite the fluorescent dye located in the environment where the metabolic gas has to be determined. The resulting fluorescence response is detected in a detector, the information is digitised, if needed the measurement is corrected (for temperature for example) and the gas concentration is calculated and displayed.

In the detailed description an example of a method according to the invention is shown wherein said change in metabolic rate denotes germination. Oxygen consumption measurements on seeds during germination and priming are important for the following reasons. With regard to quality of seed batches (both for use as plant propagation method in e.g. horticulture and in industrial applications in e.g. barley malting) the following aspects that can be achieved by measuring oxygen consumption during germination are important: (i) speed of germination, (ii) homogeneity of germination of a seed batch, (iii) monitoring system that is automated and (iv) possibility to measure large numbers of individual seeds. As the number of samples to be tested in seed companies is very large and they are currently evaluated by eye, an automated system saves a lot of work. In addition many seeds should be kept in the dark during the test, the oxygen measurements can be performed in the dark in an automated system which solves the problems with the evaluation of these types of seeds. The homogeneity of seed batches is a quality aspect of prime importance. This requires tests on individual seeds, so the possibility to automate the oxygen measurements in e.g. a measurement device using 96 wells plates offers an elegant solutions for the otherwise very labour-intensive test. During priming (this is a carefully controlled imbibition of seeds to obtain a pre-germination) it is important that the duration and extend of the priming procedure is not too long (this results in primed seeds that cannot be dried again). Monitoring of the metabolism (oxygen consumption) during the priming procedure will be an indicator of the progress of the priming that can be used to control the priming process. However, a method according to the invention is as well applicable to register a second respiratory burst as is often identified in phase 3 of germination. The invention is furthermore used for quality assurance. For example seed batches primed or germinated by different methods or under different circumstances are tested. varieties are tested on for example their germination. To be able to perform high throughput screening a method according to the invention is preferably miniaturised and/or automated. An example of such a automated/miniaturised device is depicted in Figure 7.

Within the field of seed technology, the invention provides a method to determine or monitor a rate of seed germination or development, to for example determine proper priming of seed batches.

## Detailed description

### 1. Determination of the start of seed germination by oxygen consumption

Most of conventional agriculture is engaged in growing plants from seeds. Plant breeding programs are dependent on the germination of the seeds obtained. Therefore the slow or no germination of seeds has a major impact on food production and research. The research on dormancy or environmental factors influencing the germination requires a simple method to monitor the germination process.

Methods.

One or more seeds (Triumph 1989, barley) are brought in a confined container, along with some water to induce the germination process. The container is closed. Due to the germination at a certain point in time the seed(s) will start to consume oxygen. Because the container is closed, the oxygen concentration will drop from the moment the germination starts. This can be monitored with a special oxygen sensitive coating on the inside of an optically transparent part of the container. An advantage of optical oxygen determination is the fact that the coating itself does not consume any oxygen. In this way the start of the germination can be monitored accurately. Up to now only the appearance of a root was an indication of the germination. In the experiment the first root showed after 10 to 14 hours. From the oxygen measurements we see that the germination activity showed after 3.5 hours. The oxygen consumption is an early indicator of seed germination.

The point where the germination starts can be calculated from the measured oxygen levels, as for example given in table 1. The linear extrapolation of the oxygen levels measured after 4 hours in the containers with 1, 2 and 3 seeds show an intersection with the oxygen level of an empty container at 3.5 hours after the addition of the water. This is the point in time where the metabolism of the germination starts. This is shown in figure 4. Figure 5 shows a general course of oxygen levels with seeds in a container and figure 6 an example of a calculation. With this method it is possible to examine the effect of all kind of environmental influences on the germination process. It also gives an opportunity to influence the germination process in an early stage. This method is a simple and powerful tool in germination research and in the priming of seeds.

Table 1

| Oxygen content in μg of a sample container (approx. 1 ml) with a different number of seeds. | | | |
|---|---|---|---|
| Hours | Container with 1 seed | Container with 2 seeds | Container with 3 seeds |
| 0.0 | 284 | 284 | 284 |
| 0.5 | 263 | 271 | 261 |
| 1.5 | 274 | 253 | 261 |
| 2.5 | 284 | 279 | 267 |
| 3.5 | 274 | 261 | 270 |
| 4.5 | 270 | 259 | 266 |
| 5.5 | 279 | 256 | 223 |
| 6.5 | 277 | 253 | 227 |
| 7.5 | 271 | 231 | 204 |
| 8.5 | 282 | 257 | 198 |
| 9.5 | 269 | 227 | 160 |
| 11.5 | 275 | 202 | 125 |
| 14.0 | 262 | 172 | 51 |
| 24.0 | 220 | 41 | 1 |
| 29.0 | 250 | 14 | 7 |
| 32.0 | 238 | 9 | 4 |
| 35.5 | 226 | 8 | 4 |
| 38.0 | 201 | 8 | 4 |
| 50.0 | 171 | 6 | 5 |
| 53.0 | 191 | 8 | 5 |
| 61.0 | 159 | 9 | 4 |
| 72.0 | 135 | 7 | 3 |

**2. Oxygen consumption of the roots of a rose (Roza spec.) in a confined space**

In this research application the oxygen consumption of plant roots is measured by placing the roots of a plant in a confined container with a known volume sealed hermetically around the stem in order to avoid gas exchange of the confined container and surrounding. The oxygen consumption profile of the plants under different growth conditions can be easily determined.

This example is not within the scope of the present invention. It is mentioned here to illustrate the applicability of the metabolic gas monitoring method for organisms other than seeds.

Figure 8 shows a schematic representation of the experimental set-up of a plant (for example a rose) in a confined container.

Figure 9 shows the result. In this example two different kinds of metabolisms were found, depending on the oxygen concentration.

### 3. Comparison of the start of germination between lettuce and barley seed

In this set-up the germination speed of Lettuce (Grand Rapids Ritsa) seeds and Barley (Triumph 1989) seeds was compared in several containers. The seeds were confined in small confined containers with a volume of 200 microliter. The containers were scanned for their oxygen content every 30 minutes. By means of the measured oxygen concentration profile the start of germination for each species was calculated as shown in Figure 10. The experimental conditions were identical to the germination experiment described in experiment 1.

Figure 7 shows a schematic set-up of the confined containers for this experiment. The use of small confined containers as in this example shows that a method according to the invention is easily miniaturised.

Figure 10 shows the result of the above described experiment. This result is in accordance with the visual determination of germination of the 2 tested seeds.

Figure legends

Figure 1 Measurement principle of optical oxygen sensor.

Figure 2 A rough schematic representation of a set-up for measuring metabolic gas changes and/or rates.

Figure 3 A detailed schematic representation of oxygen sensor.

Figure 4 Oxygen consumption during the germination of Triumph 1989 seeds at 25 °C.

Figure 5 Oxygen consumption during the germination of seeds.

Figure 6 Regression calculation.

Figure 7 Example of an automated version of a method according to the invention.

Figure 8 Experimental set-up for part of a plant in a confined container

Figure 9 Oxygen consumption of the roots of a Rose in a confined space

Figure 10 Determination of the start of germination by oxygen consumption in a confined space.

### References

1. Bambot S.B. et al., Phase Fluorimetric Sterilizable Optical Oxygen Sensor, Biotechnology and Bioengineering, 43:1139-1145, 1994.
2. Cox, M.E., Bunn, B., Detection of Oxygen by fluorescence quenching, Applied Optics, 24, 2114-2120, 1985
3. Holst, G.A., Flox an oxygen-flux-measuring system using a phase modulation method to evaluate the oxygen dependent fluorescent lifetime, Sensor and Actuators B 29 (1995) 231-239
4. Meier, B et al., Novel oxygen sensor material based on a ruthenium bipyridyl complex encapsulated in zeolyte Y: dramatic diffeneces in the efficience of luminescence quenching by oxygen on going from surface-adsorbed to zeolite-encapsulated fluorophores, Sensor and Actuators B 29 (1995) 240-245.
5. Marazuele, M.D. et al., Luminescence lifetime Quenching of a Ruthenimum (II) Polypyridyl Dye for Optical Sensing of Carbon Dioxide. Appl. Spectrocospy (1998), 52:1314-1320.
6. A. Draaijer, J.W. J.W. König, J.J.F. van Veen, Substraat voor het inbedden van zuurstof gevoelige kleurstof, Dutch patent application, application number 1014464
7. Hatterman-Valenti H. et al., Physiological Basis of Seed Dormancy in Woolly Cupgrass, Weed Science, 44, 87-90, 1996.
8. Chugh L.K., Sawhney S.K., Effect of Cadmium on Germination, Amylases and Rate of Respiration of Germinating Pea Seeds, Environmental Pollution, 92, 1-5, 1996.

9. Barnikol W.K.R. et al., Ein neuartiges Verfahren der Sauerstoffdetektion für Medizin, Biologie, Umweltforschung und Biotechnik auf Basis der Lumineszenzlöschung, Biomedizinische Technik, 41, 170-177, 1996.

**Claims**

1. A method for determining the metabolic rate or a change therein of an individual seed comprising placing said seed in a container and measuring the concentration of oxygen in said container by determining the fluorescence quenching of a fluorescent dye present in said container to measure consumption of said oxygen by said seed.

2. A method according to claim 1 wherein said metabolic rate or change in metabolic rate denotes germination.

3. A method according to claim 1 or 2, wherein said dye is present in a gas permeable compound.

4. A method according to claim 3, wherein said compound comprises a hydrophobic polymer.

5. A method according to anyone of claims 1 to 4 wherein said dye is present in at least a part of an inner coating of said container.

6. A method to determine or monitor a rate of seed development comprising using of a method according to any of claims 1 to 5.

7. A method to determine homogeneity of a seed batch wherein individual seeds from said batch are tested according to a method according to any of claims 1 to 6.

8. A method to determine seed quality of a seed batch wherein individual seeds from said batch are tested according to a method according to claim 1 to 7.

9. A method according to claim 7 or 8 wherein the individual seeds are placed in a 96-wells plate.

**Patentansprüche**

1. Verfahren zur Bestimmung der metabolischen Rate eines einzelnen Samens oder einer Veränderung derselben, bei dem man den Samen in einen Behälter einbringt und die SauerstoffKonzentration in dem Behälter durch Bestimmung des Fluoreszenz-Quenchens eines in dem Behälter vorliegenden Fluoreszenzfarbstoffs misst, um den Verbrauch des Sauerstoffs durch den Samen zu messen.

2. Verfahren nach Anspruch 1, **dadurch gekennzeichnet, dass** die metabolische Rate oder die Veränderung der metabolischen Rate die Keimung anzeigt.

3. Verfahren nach Anspruch 1 oder 2, **dadurch gekennzeichnet, dass** der Farbstoff in einer gasdurchlässigen Verbindung vorliegt.

4. Verfahren nach Anspruch 3, **dadurch gekennzeichnet, dass** die Verbindung ein hydrophobes Polymer umfasst.

5. Verfahren nach einem der Ansprüche 1 bis 4, **dadurch gekennzeichnet, dass** der Farbstoff zumindest in einem Teil der inneren Beschichtung des Behälters vorliegt.

6. Verfahren zur Bestimmung oder zum Verfolgen einer Keimentwicklungsrate, bei dem man ein Verfahren nach einem der Ansprüche 1 bis 5 verwendet.

7. Verfahren zur Bestimmung der Homogenität einer Samenmenge, **dadurch gekennzeichnet, dass** einzelne Samen der Menge nach einem Verfahren nach einem der Ansprüche 1 bis 6 untersucht werden.

8. Verfahren zur Bestimmung der Samenqualität einer Samenmenge, **dadurch gekennzeichnet, dass** einzelne Samen der Menge nach einem Verfahren nach einem der Ansprüche 1 bis 7 untersucht werden.

9. Verfahren nach Anspruch 7 oder 8, **dadurch gekennzeichnet, dass** die einzelnen Samen in eine Platte mit 96 Vertiefungen eingebracht werden.

## Revendications

1. Procédé pour déterminer le taux métabolique ou un changement de celui-ci d'une graine individuelle comportant le placement de ladite graine dans un conteneur et la mesure de la concentration en oxygène dans ledit conteneur en déterminant l'extinction de fluorescence d'un colorant fluorescent présent dans ledit conteneur pour mesurer la consommation dudit oxygène par ladite graine.

2. Procédé selon la revendication 1, dans lequel ledit taux métabolique ou changement de taux métabolique désigne la germination.

3. Procédé selon la revendication 1 ou 2, dans lequel ledit colorant est présent dans un composé perméable aux gaz.

4. Procédé selon la revendication 3, dans lequel ledit composé comporte un polymère hydrophobe.

5. Procédé selon l'une quelconque des revendications 1 à 4, dans lequel ledit colorant est présent dans au moins une partie d'un revêtement intérieur dudit conteneur.

6. Procédé pour déterminer ou surveiller un taux de développement de graine comportant l'utilisation d'un procédé selon l'une quelconque des revendications 1 à 5.

7. Procédé pour déterminer l'homogénéité d'un lot de graines, dans lequel des graines individuelles provenant dudit lot sont testées conformément à un procédé selon l'une quelconque des revendications 1 à 6.

8. Procédé pour déterminer une qualité de graine d'un lot de graines, dans lequel des graines individuelles provenant dudit lot sont testées conformément à un procédé selon les revendications 1 à 7.

9. Procédé selon la revendication 7 ou 8, dans lequel les graines individuelles sont placées dans une plaque de 96 puits.

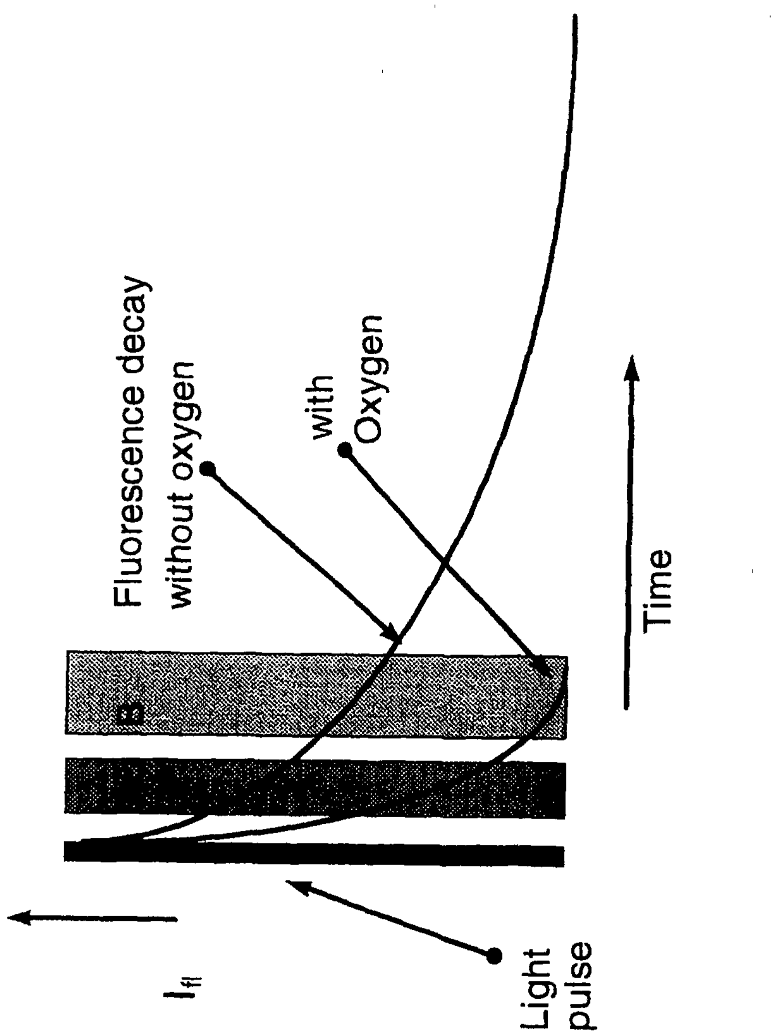
Fluorescence decay
without oxygen
with
Oxygen
B
$I_{fl}$
Light
pulse
Time

Fig. 1

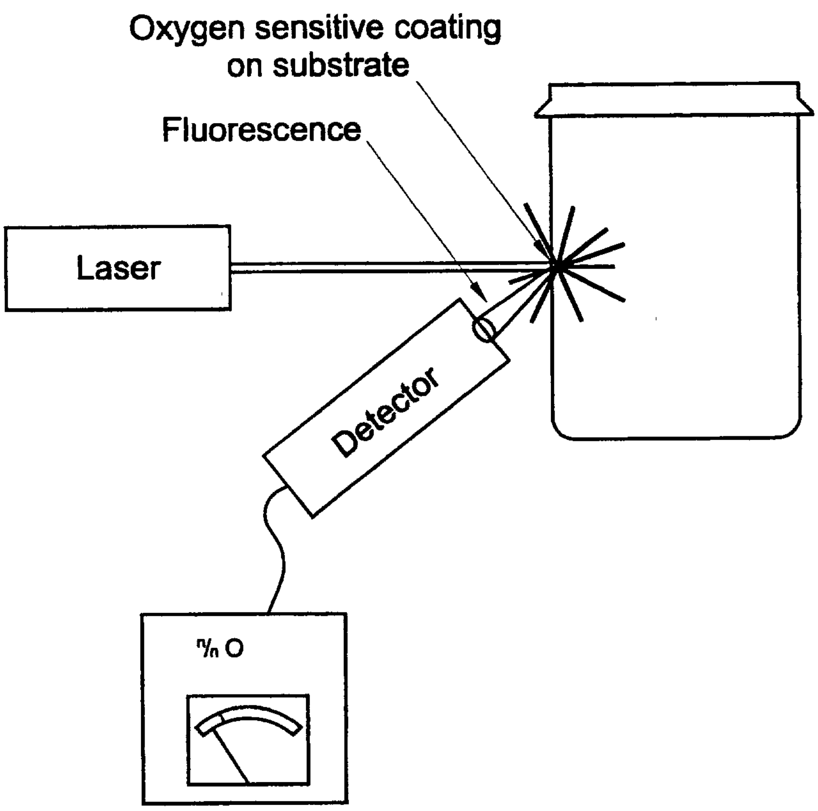
Oxygen sensitive coating on substrate
Fluorescence
Laser
Detector
% O

Fig. 2

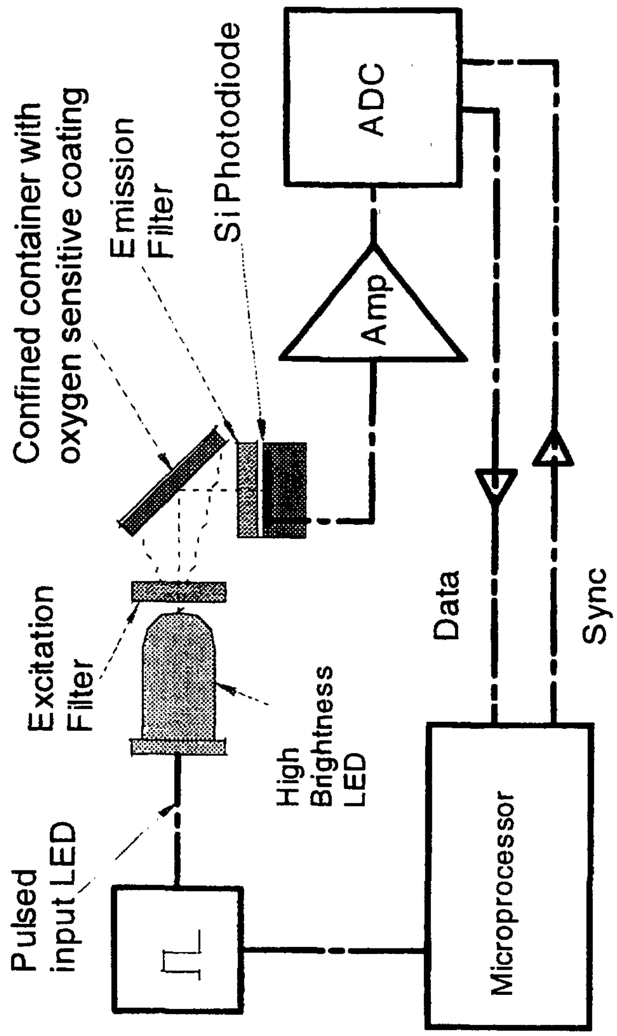
Pulsed input LED
Excitation Filter
High Brightness LED
Confined container with oxygen sensitive coating
Emission Filter
Si Photodiode
Amp
ADC
Data
Sync
Microprocessor

Fig. 3

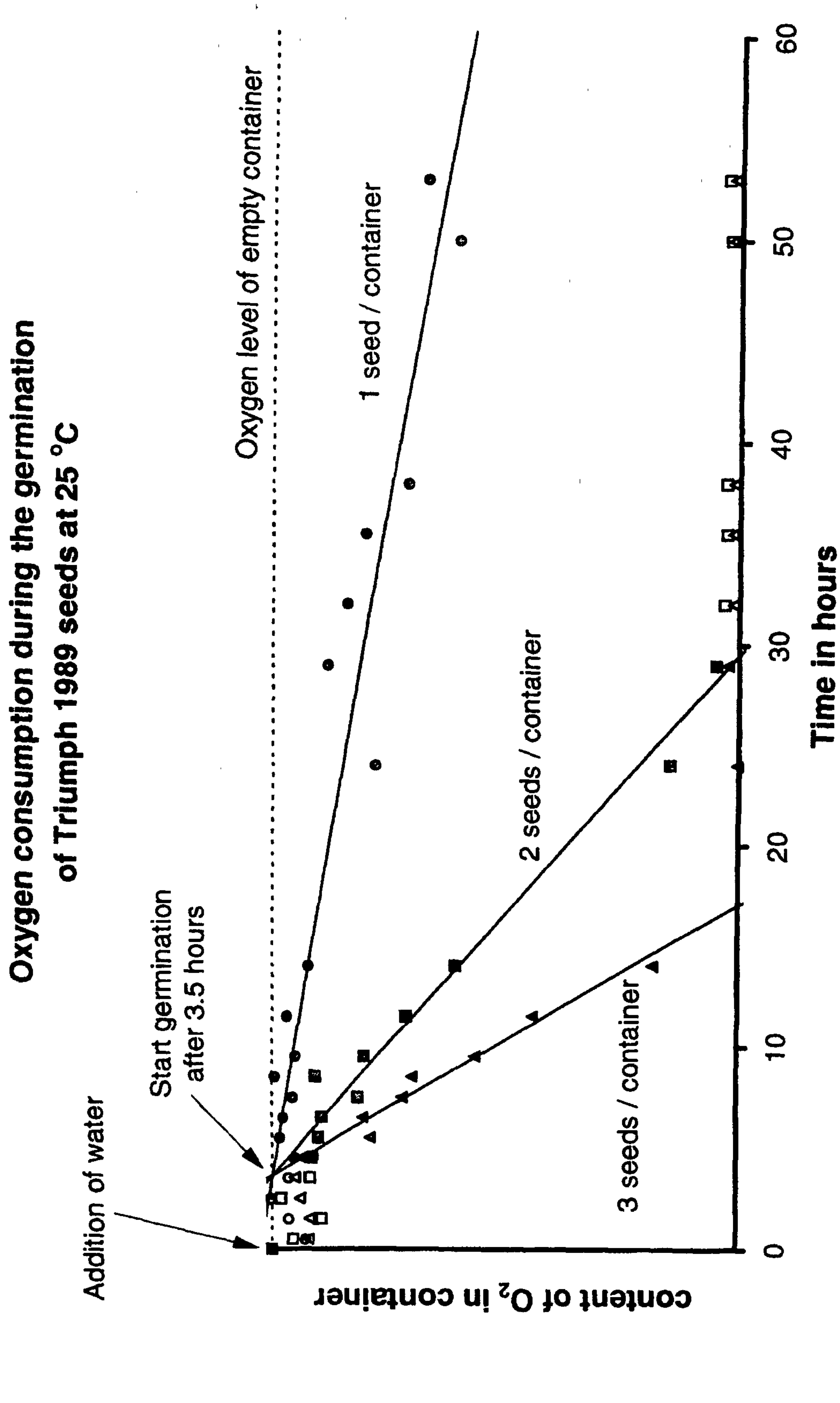
Oxygen consumption during the germination
of Triumph 1989 seeds at 25 °C
Addition of water
Start germination
after 3.5 hours
Oxygen level of empty container
1 seed / container
2 seeds / container
3 seeds / container
content of $O_2$ in container
Time in hours
0
10
20
30
40
50
60

Fig. 4

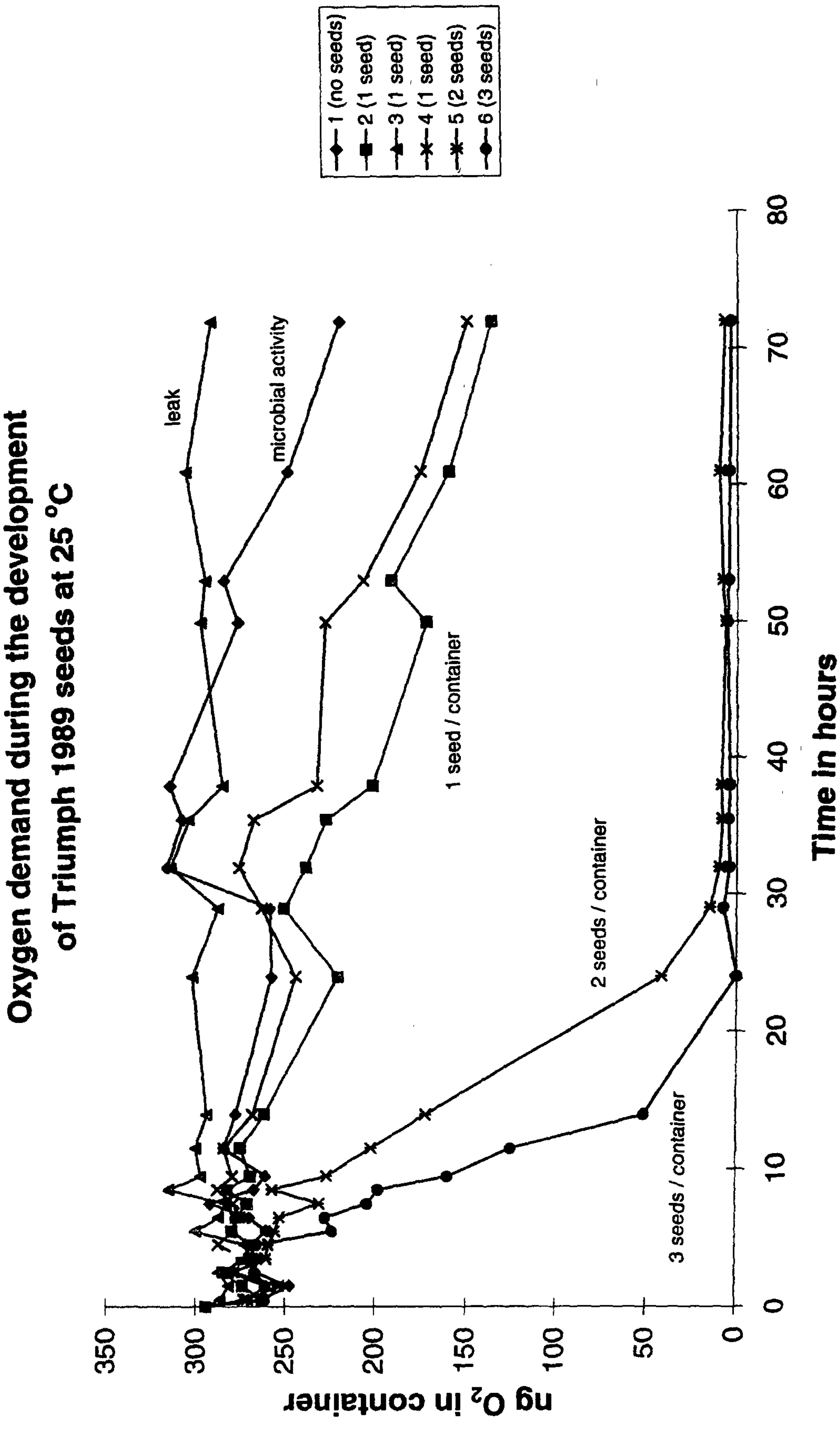
Oxygen demand during the development
of Triumph 1989 seeds at 25 °C
1 (no seeds)
2 (1 seed)
3 (1 seed)
4 (1 seed)
5 (2 seeds)
6 (3 seeds)
leak
microbial activity
1 seed / container
2 seeds / container
3 seeds / container
ng $O_2$ in container
350
300
250
200
150
100
50
0
0
10
20
30
40
50
60
70
80
Time in hours

Fig. 5

| Hours | 2 (1 seed) | 4 (1 seed) | 5 (2 seeds) | 6 (3 seeds) |
|---|---|---|---|---|
| [redacted] | | | | |
| 6.5 | 277 | 275 | 253 | 227 |
| 7.5 | 271 | 279 | 231 | 204 |
| 8.5 | 282 | 287 | 257 | 198 |
| 9.5 | 269 | 279 | 227 | 160 |
| 11.5 | 275 | 284 | 202 | 125 |
| 14.0 | 262 | 268 | 172 | |
| 24.0 | 220 | 244 | | |
| 29.0 | 250 | 263 | | |
| 32.0 | 238 | 275 | | |
| 35.5 | 226 | 267 | | |
| 38.0 | 201 | 231 | | |

| Hours | 2 (1 seed) | 4 (1 seed) | 5 (2 seeds) | 6 (3 seeds) |
|---|---|---|---|---|
| 5.5 | 0 | 0 | 23 | 56 |
| 6.5 | 2 | 4 | 26 | 52 |
| 7.5 | 8 | 0 | 48 | 75 |
| 8.5 | -3 | -8 | 22 | 81 |
| 9.5 | 10 | 0 | 52 | 119 |
| 11.5 | 4 | -5 | 77 | 154 |
| 14.0 | 17 | 11 | 107 | |
| 24.0 | 59 | 35 | | |
| 29.0 | 29 | 16 | | |
| 32.0 | 41 | 4 | | |
| 35.5 | 53 | 12 | | |
| 38.0 | 78 | 48 | | |

| | | | | |
|---|---|---|---|---|
| r.c. | 2.001 | 0.950 | 9.948 | 17.747 |
| intercept | -12.083 | -7.808 | -38.721 | -55.444 |
| start hour | 6.0 | 8.2 | 3.9 | 3.1 |

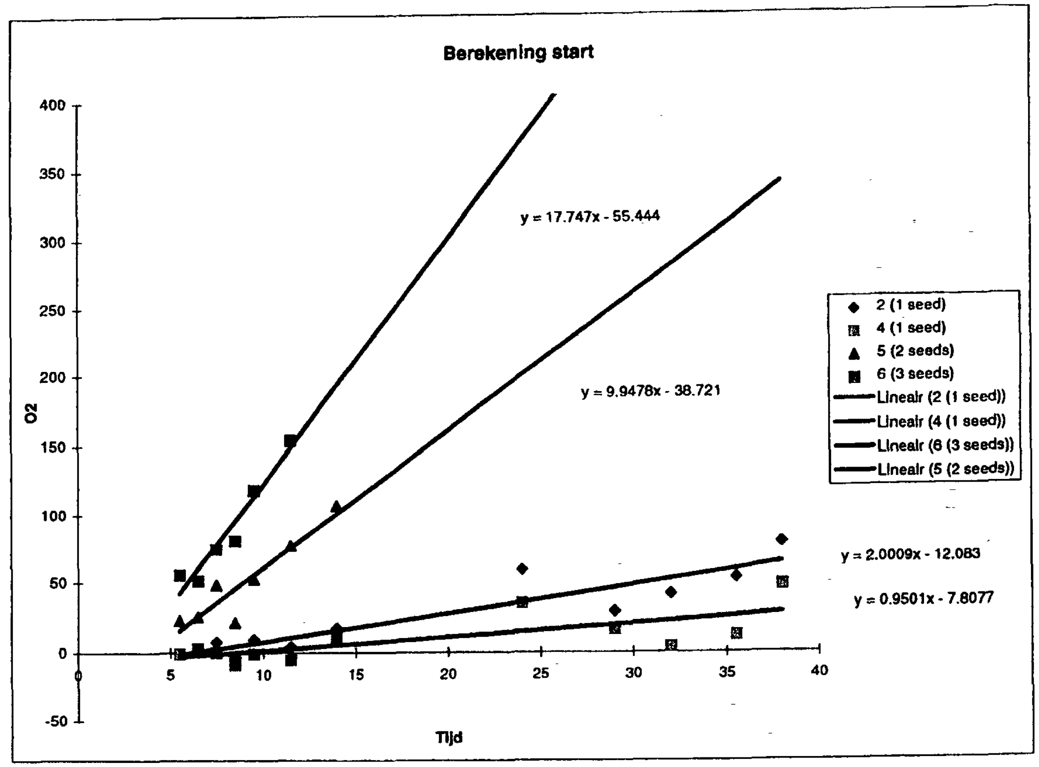


Fig. 6

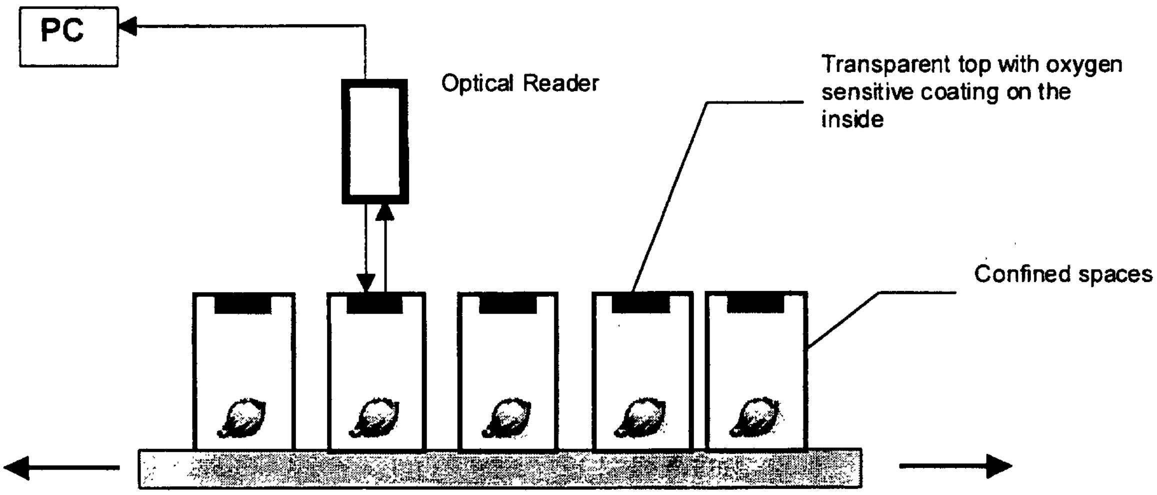
PC
Optical Reader
Transparent top with oxygen sensitive coating on the inside
Confined spaces

Fig. 7

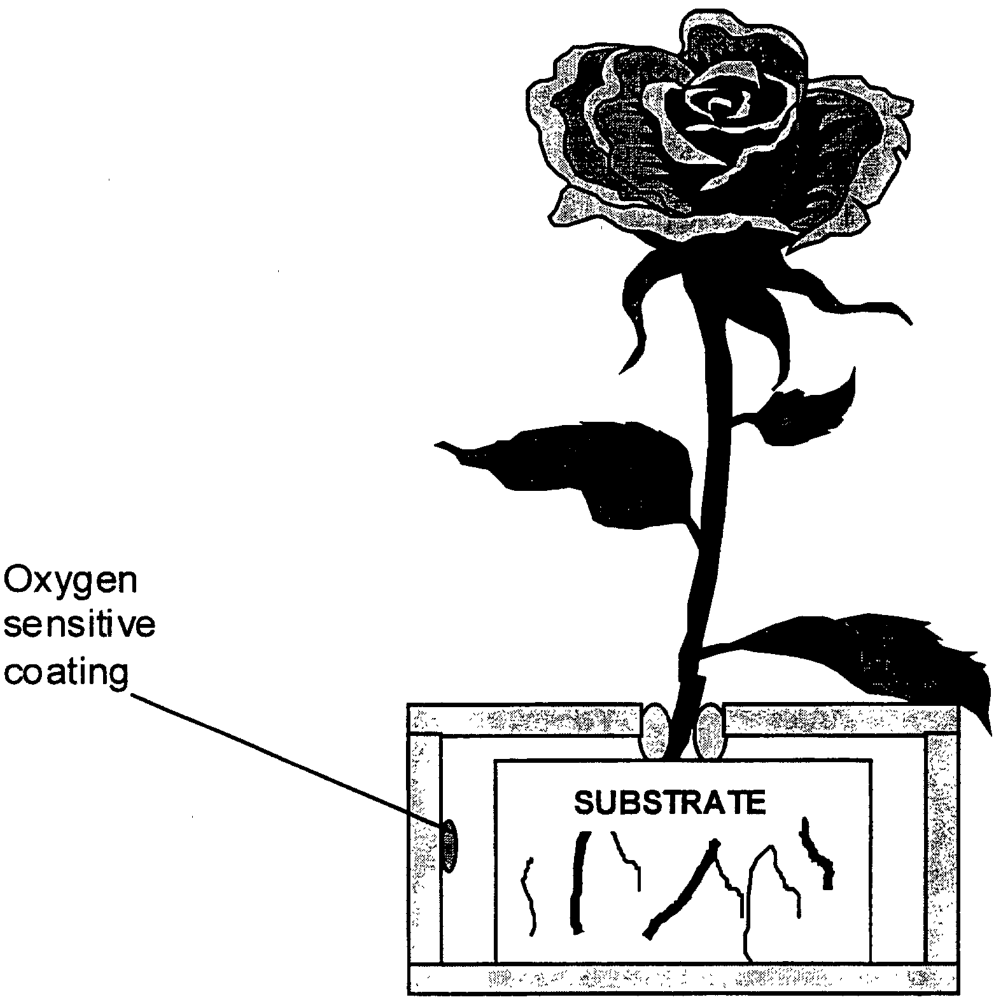


Fig. 8

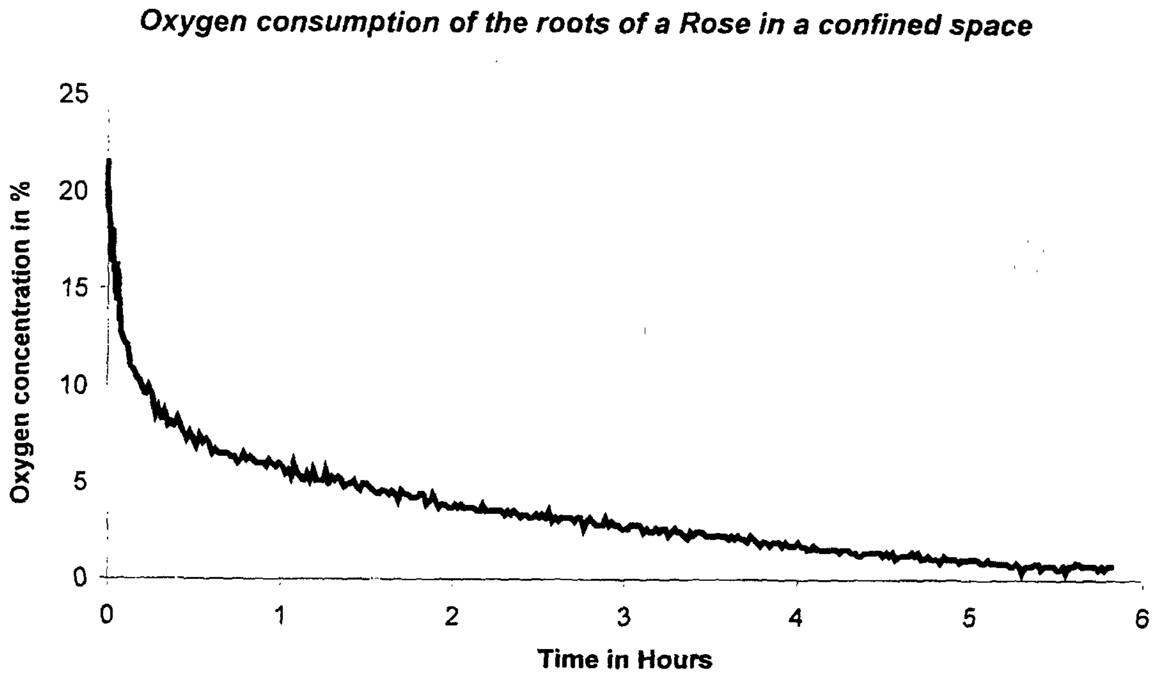
Oxygen consumption of the roots of a Rose in a confined space
Oxygen concentration in %
25
20
15
10
5
0
0
1
2
3
4
5
6
Time in Hours

Fig. 9

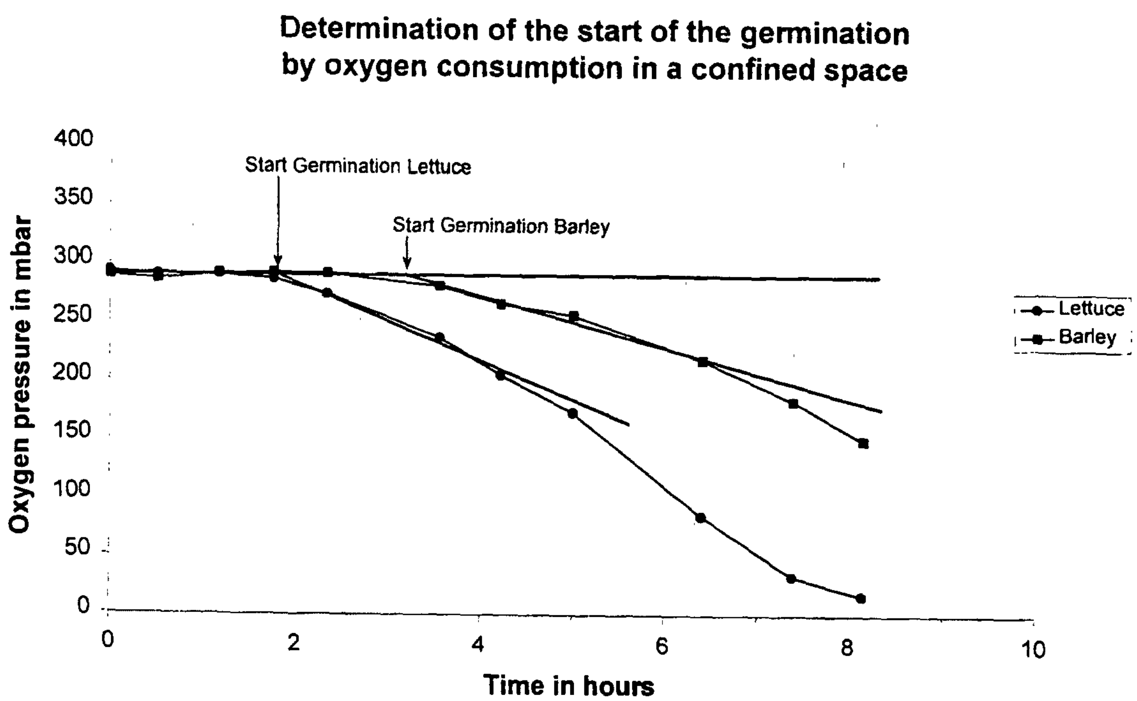
Determination of the start of the germination
by oxygen consumption in a confined space
Start Germination Lettuce
Start Germination Barley
Oxygen pressure in mbar
400
350
300
250
200
150
100
50
0
0
2
4
6
8
10
Time in hours
Lettuce
Barley

Fig. 10